(19) Europäisches Patentamt
European Patent Office
Office européen des brevets

(11) **EP 1 818 042 A2**

(12) # DEMANDE DE BREVET EUROPEEN

(43) Date de publication:
**15.08.2007 Bulletin 2007/33**

(51) Int Cl.:
*A61K 8/26* (2006.01)   *A61K 8/25* (2006.01)
*A61Q 3/02* (2006.01)   *A61K 8/89* (2006.01)

(21) Numéro de dépôt: **07101599.4**

(22) Date de dépôt: **01.02.2007**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **13.02.2006 FR 0650509**

(71) Demandeur: **L'Oréal**
**75008 Paris (FR)**

(72) Inventeurs:
• **Puisset, Virginie**
**75005, PARIS (FR)**

• **Guerchet, Laurence**
**94400, VITRY SUR SEINE (FR)**
• **Coffey-Dawe, Lizabeth-Anne**
**93600, AULNAY SOUS BOIS (FR)**

(74) Mandataire: **Duvert, Sandra et al**
**L'ORÉAL**
**DIPI**
**River PLaza**
**25-29 Quai Aulagnier**
**92600 Asnieres-sur-Seine (FR)**

(54) **Vernis à ongle de texture gélifiée**

(57) La présente invention a pour objet un vernis à ongles comprenant un milieu cosmétiquement acceptable et au moins un agent épaississant thixotrope, ladite composition présentant une viscosité à 25°C d'au moins 0,6 Pa.s.

La présente invention a également pour objet un procédé cosmétique de maquillage des ongles consistant à abaisser la viscosité d'une composition de vernis à ongles présentant une viscosité à 25°C d'au moins 0,6 Pa.s à l'aide d'une action non chimique, en particulier mécanique, simultanément ou préalablement à l'application sur les ongles de ladite composition.

**EP 1 818 042 A2**

Printed by Jouve, 75001 PARIS (FR)

## Description

La présente invention concerne un vernis à ongles de viscosité élevée et de texture gélifiée ainsi qu'un procédé de revêtement des ongles.

La composition de vernis à ongles, colorée ou transparente, peut être employée comme base pour vernis ou "base-coat" en terminologie anglo-saxonne, comme produit de maquillage des ongles, comme composition de finition, encore appelée "top-coat", à appliquer sur le produit de maquillage des ongles ou bien encore comme produit de soin cosmétique des ongles.

Les vernis à ongles conventionnels se présentent sous forme liquide ou fluide et sont généralement conditionnés dans des flacons. Ils comprennent en général des particules solides telles que les pigments, les nacres, les charges, qui sont en dispersion dans le milieu continu aqueux ou le milieu solvant organique de la composition.
Cette forme fluide impose une bonne dispersion des pigments de manière à préserver l'homogenéité de la couleur du vernis liquide ainsi que du film de vernis une fois appliqué sur les ongles.
Or ces particules ont tendance à sédimenter au cours du temps, du fait de leur densité qui est supérieure à celle du milieu continu dans lequel elles sont dispersées. Cette sédimentation se traduit par une modification de l'aspect macroscopique de la composition, et en particulier, dans le cas de vernis à ongles colorés, par une hétérogénéité de la couleur du vernis.

On cherche donc à obtenir des vernis à ongles présentant une bonne dispersion des particules, en particulier des pigments, et donc une bonne stabilité et une bonne homogénéité de la couleur dans le temps, ainsi qu'un film déposé sur les ongles ayant une couvrance satisfaisante.
En outre d'un point de vue pratique, il serait intéressant de disposer de vernis présentant de nouvelles textures qui soient faciles à manipuler (sans problèmes de renversement ou de goutte)

La demanderesse a constaté que ces avantages pouvaient être obtenus par la mise en oeuvre d'une composition de vernis à ongles se présentant sous forme non liquide, de haute viscosité et de texture gélifiée permettant une dispersion homogène des pigments, ladite composition présentant un comportement thixotrope.
De plus cette texture gélifiée permet une meilleure organisation et orientation des particules colorantes (en particulier des nacres) dans la composition lors de son application sur les ongles puis lors du séchage du film de vernis, permettant ainsi d'obtenir un effet coloriel et une brillance supérieurs aux films issus de vernis à ongles fluides conventionnels dans lesquels les particules colorantes ne suivent pas une orientation préférentielle.

En outre, cette composition, contrairement aux vernis à ongles conventionnels, ne coule pas, ne goutte pas et permet d'obtenir, après application sur les ongles, un film qui sèche rapidement tout en étant homogène et lisse; et qui présente de bonnes propriétés de tenue et de brillance.

La présente invention a donc pour objet une composition de vernis à ongles comprenant un milieu cosmétiquement acceptable et au moins un agent épaississant thixotrope, ladite composition présentant une viscosité à 25˚C d'au moins 0,6 Pa.s.
En particulier, la nature et/ou la quantité dudit agent épaississant sont telle(s) que, en réponse à une action non chimique, notamment mécanique, préalablement ou simultanément à l'application de la composition sur les ongles, la viscosité de la composition peut être abaissée de manière réversible à une valeur n'excédant pas 0,4 Pa.s, de préférence à une valeur n'excédant pas 0,3 Pa.s.

### Mesure de la viscosité

La viscosité de la composition est mesurée à 25˚C à l'aide d'un Rhéomat 180 (Société LAMY) équipé d'un mobile MS-R1, MS-R2, MS-R3, MS-R4 ou MS-R5 choisi en fonction de la consistance de la composition, tournant à une vitesse de rotation de 200 t.min-1. La mesure est prise après 10 min de rotation. Les mesures de viscosité sont réalisées au maximum 1 semaine après fabrication.

Le vernis à ongles de l'invention peut présenter une viscosité allant de 0,6 à 20 Pa.s, de préférence de 0,7 à 15 Pa.s et, mieux de 0,75 à 10 Pa.s.

L'invention a encore pour objet un procédé cosmétique de maquillage des ongles consistant à abaisser la viscosité d'une composition de vernis à ongles présentant une viscosité à 25˚C d'au moins 0,6 Pa.s à l'aide d'une action non chimique, notamment mécanique, simultanément ou préalablement à l'application sur les ongles de ladite composition.

L'invention a également pour objet une composition de vernis à ongles comprenant un milieu cosmétiquement acceptable et au moins un agent épaississant thixotrope, ladite composition présentant un caractère thixotropique.

L'invention a également pour objet une composition de vernis à ongles comprenant un milieu cosmétiquement acceptable et au moins un agent épaississant thixotrope, ladite composition présentant un caractère rhéofluidifiant.

L'invention a aussi pour objet l'utilisation d'une composition telle que définie ci-dessus pour obtenir un film de vernis déposé sur les ongles, homogène et présentant de bonnes propriétés de brillance et/ou une bonne couvrance.

L'invention a aussi pour objet l'utilisation d'une composition telle que définie ci-dessus pour obtenir un film de vernis déposé sur les ongles qui sèche rapidement .

L'invention a encore pour objet un procédé cosmétique de maquillage des ongles consistant à abaisser la viscosité d'une composition de vernis à ongles présentant une viscosité à 25°C d'au moins 0,6 Pa.s à l'aide d'une action non chimique, notamment mécanique, simultanément ou préalablement à l'application sur les ongles de ladite composition.

L'invention a enfin pour objet l'utilisation d'une quantité suffisante d'au moins un agent épaississant thixotrope pour obtenir une composition de vernis à ongles stable présentant une viscosité à 25°C d'au moins 0,6 Pa.s.

Par milieu cosmétiquement acceptable, on entend un milieu non toxique et susceptible d'être appliqué sur les matières kératiniques d'êtres humains, en particulier les ongles.

La composition selon l'invention présente avantageusement un caractère thixotrope.

Par composition ayant un caractère thixotrope, ou composition thixotrope, on entend, au sens de l'invention, une composition structurée qui fluidifie (notamment sa viscosité diminue) lorsqu'on lui applique une action non chimique, en particulier mécanique, et qui récupère toute ou partie de sa viscosité initiale après un temps de repos suffisant qui peut être plus ou moins long, à température ambiante.

En particulier, la composition a les propriétés suivantes :

- la composition a un caractère rhéofluidifiant, c'est-à-dire que la viscosité de la composition diminue lorsque l'on applique à la composition des cisaillements croissants ;
- la composition après l'application d'un cisaillement intense se fluidifie (notamment sa viscosité diminue). La viscosité, la consistance et l'élasticité de la composition, après sa destructuration, en particulier après un temps de repos d'une minute après avoir appliqué le cisaillement, sont inférieures à celles de la composition avant l'application du cisaillement intense ;
- la composition régénère partiellement ou totalement sa structure initiale après un temps de repos suffisant et la restructuration de la composition est retardée dans le temps.. La restructuration de la composition ne se produit donc pas instantanément mais de façon différée dans le temps.

  Une définition de composition thixotrope est notamment indiquée dans l'ouvrage "Comprendre la rhéologie - De la circulation du sang à la prise du béton" de P. Coussot et J.L. Grossiord, EDP Science, 2002, pages 16 et 17.

Le comportement thixotrope de la composition peut notamment être caractérisé via les mesures de viscosité de la composition sous faible vitesse cisaillement puis sous forte vitesse de cisaillement, comme décrit ci-après :

Ces mesures sont effectuées sur un rhéomètre à contrainte imposée, Haake RheoStress® RS 600 de la société ThermoRhéo, équipé d'un bain thermostaté et d'un mobile en acier inoxydable à géométrie cône/plan, de diamètre 35 mm et d'angle 2˚, avec un entrefer de 0,104 mm. Les 2 surfaces sont « sablées » pour limiter les phénomènes de glissement aux parois. Un dispositif anti-évaporation (cloche à solvants) est utilisé.

Les mesures sont effectuées à 20°C $\pm$ 1°C.

Dans un premier temps, l'échantillon est mis en température à 20°C $\pm$ 1°C pendant 300 secondes (sans aucun cisaillement appliqué).

a) On applique des contraintes croissantes à l'échantillon en partant d'une contrainte initiale de 0 Pa pour arriver à une contrainte finale de 500 Pa, les contraintes n'étant appliquées qu'une seule fois (ce qui correspond à une rampe en cisaillement allant de $10^{-1}$ s$^{-1}$ à 400 s$^{-1}$). On trace alors l'évolution de la viscosité η en fonction du gradient de cisaillement $\dot{\gamma}$. On prend la mesure sur 40 points répartis de façon logarithmique.

On attend l'obtention d'une valeur stable entre chaque contrainte, le temps d'attente entre chaque contrainte étant de 30 s.

b) On destructure ensuite la composition en lui appliquant un cisaillement continu correspondant à une contrainte de 500Pa (correspondant à un cisaillement de 400 s$^{-1}$), pendant 30 secondes.

c) On applique à l'échantillon des contraintes décroissantes en partant d'une contrainte initiale de 500 Pa pour arriver à une contrainte finale de 0 Pa, les contraintes n'étant appliquées qu'une seule fois.

On prend la mesure sur 40 points répartis de façon logarithmique.

On attend l'obtention d'une valeur stable entre chaque contrainte, le temps d'attente entre chaque contrainte étant de 30 s.

La gamme de gradient de cisaillement va de 400 s$^{-1}$ à $10^{-3}$ s$^{-1}$. Dans la gamme considérée, la valeur maximale de 400 s$^{-1}$ doit être prise en compte avec une incertitude de mesure de $\pm$ 10 s$^{-1}$

On trace alors l'évolution de la viscosité η en fonction du gradient de cisaillement $\dot{\gamma}$.

d) L'échantillon est ensuite soumis à une contrainte imposée de 10 Pa pendant 1000 s, et on mesure l'évolution de la viscosité en fonction du temps.

e) On applique à nouveau des contraintes croissantes à l'échantillon en partant d'une contrainte initiale de 0 Pa pour arriver à une contrainte finale de 500 Pa, les contraintes n'étant appliquées qu'une seule fois (ce qui correspond à un gradient de cisaillement allant de $10^{-2}$ s $^{-1}$ à 300 s$^{-1}$).

L'analyse des résultats se fait au travers de la représentation graphique de l'évolution de la viscosité, notée $\eta$, en fonction du gradient de cisaillement, noté $\dot{\gamma}$. La viscosité $\eta$, le gradient de cisaillement $\dot{\gamma}$ et la contrainte imposée $\tau$ étant reliés entre eux par la relation

$$\dot{\gamma} = \frac{\tau}{\eta}$$

La composition, est telle qu'elle présente une viscosité, telle que mesurée au cours de l'étape e), à une vitesse de cisaillement de $4.10^{-2}$ s$^{-1}$, allant de $10^2$ à $10^4$ Pa.s, mieux de $5.10^2$ à $5.10^3$ Pa.s, encore mieux de 600 à 4000 Pa.s.

Le caractère rhéofluidifiant de la composition est notamment caractérisé par une différence (viscosité mesurée à l'étape e), à une vitesse de cisaillement de 100 s$^{-1}$ - viscosité mesurée mesurée à l'étape e), à une vitesse de cisaillement de $4.10^{-2}$ s$^{-1}$), allant de 10 à $10^5$ Pa.s, mieux de $10^2$ à $10^4$ Pa.s.

Le comportement thixotrope de la composition est caractérisé par une différence de viscosité mesurée à une vitesse de cisaillement de 1 s$^{-1}$ entre l'étape c) et l'étape e) (viscosité mesurée au cours de l'étape c) à une vitesse de cisaillement de 1 s$^{-1}$ - viscosité mesurée au cours de l'étape e) d'au moins 1 Pa .s, de préférence d'au moins 10 Pa.s, mieux d'au moins 20 Pa.s, encore mieux d'au moins 30 Pa.s, préférentiellement d'au moins 40 Pa.s.

En particulier, la différence de viscosité mesuré à une vitesse de cisaillement de 1 s$^{-1}$ entre l'étape c) et l'étape e) soit la différence (viscosité mesurée au cours de l'étape c) à une vitesse de cisaillement de 1 s$^{-1}$ - viscosité mesurée au cours de l'étape e) allant de 1 à 1000 Pa.s, mieux de 20 à 500 Pa.s, en particulier de 40 à 200 Pa.s.

*Mesure des caractéristiques viscoélastiques*

Les compositions conformes à l'invention ont avantageusement un comportement viscoélastique, à caractère élastique dominant.

De façon générale, un matériau est dit viscoélastique quand, sous l'effet du cisaillement, il possède à la fois les caractéristiques d'un matériau élastique, c'est à dire capable de stocker de l'énergie et les caractéristiques d'un matériau visqueux, c'est à dire capable de dissiper de l'énergie.

Plus particulièrement, le comportement viscoélastique des compositions conformes à l'invention peut être caractérisé par son module de rigidité G*, son élasticité $\delta$ et son seuil d'écoulement $\tau_c$ ; ces paramètres sont notamment définis dans l'ouvrage « Initiation à la rhéologie », G. Couarraze et J.L. Grossiord, 2ème édition, 1991, Edition Lavoisier-Tec 1 Doc.

Les mesures sont effectuées sur un rhéomètre à contrainte imposée, « Haake RheoStress 600® » de la société ThermoRhéo, équipé d'un bain thermostaté et d'un mobile en acier inoxydable à géométrie plan/plan, le plan ayant un diamètre de 20 mm et un entrefer (distance entre le plan inférieur - appelé plan stator - sur lequel est déposée la composition et le plan supérieur - appelé plan rotor) de 1 mm. Les 2 plans sont striés pour limiter les phénomènes de glissement aux parois des plans. Un dispositif anti-évaporation (cloche à solvants) est utilisé.

Les mesures sont effectuées à 20˚C $\pm$ 1˚C.

La composition est soumise à un cisaillement harmonique selon une contrainte $\tau(t)$ variant de façon sinusoïdale selon une pulsation $\omega$ ($\omega = 2\Pi\nu$), $\nu$ étant la fréquence du cisaillement appliqué.

La contrainte $\tau(t)$ et la déformation $\gamma(t)$ sont définies respectivement par les relations suivantes :

$$\tau(t) = \tau_0 \cos(\omega \cdot t) \qquad \gamma(t) = \gamma_0 \cos(\omega \cdot t - \delta)$$

$\tau_0$ étant l'amplitude maximale de la contrainte et $\gamma_0$ étant l'amplitude maximale de la déformation. $\delta$ est l'angle de déphasage entre la contrainte et la déformation et G* correspond au rapport $\tau_0$ sur $\gamma_0$.

Les mesures sont effectuées à une fréquence fixe de 1 Hz ($\nu$= 1 Hz).

Dans un premier temps, l'échantillon est mis en température à 20˚C $\pm$ 1˚C pendant 300 secondes.

On applique ensuite des contraintes croissantes à l'échantillon en partant d'une contrainte initiale égale à 0,01 Pa pour arriver à une contrainte finale de 1000 Pa, pour aller jusqu'à la destruction de l'échantillon (les contraintes n'étant appliquées qu'une seule fois).

On mesure l'évolution du module de rigidité G* (correspondant au rapport de $\tau_o$ sur $\gamma_o$.) et le déphasage $\delta$ (correspondant à l'angle de déphasage de la contrainte appliquée par rapport à la déformation mesurée) en fonction de la contrainte $\tau$ (t) appliquée.

On mesure en particulier la déformation de la composition pour la zone de contrainte dans laquelle la variation du module de rigidité G* et de l'élasticité $\delta$ est inférieure à 7 % (zone des microdéformations) et on détermine ainsi les paramètres dits « plateaux » Gp et $\delta_p$.

La contrainte seuil $\tau_c$ (la composition ne s'écoulant pas sous son propre poids, la contrainte seuil correspondant à la force minimale qu'il est nécessaire d'appliquer à la composition pour provoquer son écoulement) est déterminée à partir de la courbe G* = f($\tau$). On la définit comme la valeur de $\tau$ à l'intersection des 2 tangentes à la courbe G*=f($\tau$) pour les faibles valeurs de $\tau$ et pour les fortes valeurs de $\tau$.

Le comportement viscoélastique des compositions selon l'invention est notamment caractérisé par un module de rigidité plateau Gp supérieur à 100 Pa, de préférence supérieur à 500 Pa.

C'est pourquoi l'invention a encore pour objet une composition de vernis à ongles comprenant un milieu cosmétiquement acceptable et au moins un agent épaississant thixotrope, ladite composition présentant un module de rigidité plateau Gp supérieur à 100 Pa.

En particulier, les compositions selon l'invention possèdent un module de rigidité plateau Gp allant de 100 à $2.10^6$ Pa.s, de préférence de $5.10^2$ à $10^4$ Pa.s, mieux de 800 à 4000 Pa.s, et en particulier de 1000 à 3000 Pa.s.

Les compositions conformes à l'invention peuvent par ailleurs présenter une élasticité $\delta_p$ allant de 2˚ à 30˚, et en particulier allant de 15˚ à 25˚ et un seuil d'écoulement $\tau_c$ allant de 10 Pa à $3.10^4$ Pa, et en particulier allant de 30 Pa à 500 Pa et mieux de 50 à 200 Pa.

Agent épaississant

La composition selon l'invention comprend un agent épaississant thixotrope en une quantité suffisante pour conférer à la composition une viscosité au repos suffisante pour lui donner sa texture, et un comportement thixotrope. En particulier, la nature et/ou la quantité dudit agent épaississant sont telle(s) que, en réponse à une action non chimique, notamment mécanique, préalablement ou simultanément à l'application de la composition sur les ongles, la viscosité de la composition peut être abaissée de manière réversible à une valeur n'excédant pas 0,4 Pa.s, de préférence à une valeur n'excédant pas 0,3 Pa.s.

L'agent épaississant thixotrope peut être présent en une teneur supérieure ou égale à 1,7% en poids, allant par exemple de 1,7 % à 15 % en poids, par rapport au poids total de la composition, de préférence supérieure ou égale à 2% en poids, allant par exemple de 2 % à 10% en poids, préférentiellement allant de 2 % à 7,5 % en poids.

L'agent épaississant peut être choisi parmi les argiles hydrophiles ou organophiles, les silices pyrogénées hydrophiles ou hydrophobes, les organopolysiloxanes élastomériques et leurs mélanges.

Les argiles sont des silicates contenant un cation pouvant être choisi parmi les cations de calcium, de magnésium, d'aluminium, de sodium, de potassium, de lithium et leurs mélanges. On entend par argile hydrophile une argile apte à gonfler dans l'eau ; cette argile gonfle dans l'eau et forme après hydratation une dispersion colloïdale.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites. Ces argiles peuvent être d'origine naturelle ou synthétique.

Comme argile hydrophile , on peut citer les smectites telles que les saponites, les hectorites, les montmorillonites, les bentonites, la beidellite et en particulier les hectorites synthétiques (appelées aussi laponites) comme les produits vendus par la société Laporte sous le nom Laponite XLG, Laponite RD, Laponite RDS (ces produits sont des silicates de sodium et de magnésium et en particulier des silicates de sodium, de lithium et de magnésium) ; les bentonites comme le produit vendu sous la dénomination Bentone HC par la société RHEOX ; les silicates de magnésium et d'aluminium notamment hydratés comme les produits vendus par la société Vanderbilt Company sous le nom Veegum ultra, Veegum HS, Veegum DGT, ou encore les silicates de calcium et notamment celui sous forme synthétique vendu par la société sous le nom de Micro-cel C. Les argiles organophiles sont des argiles modifiées par des composés chimiques rendant l'argile apte à gonfler dans les milieux solvants.

L'argile peut être choisie parmi la montmorrillonite, la bentonite, l'hectorite, l'attapulgite, la sépiolite , et leurs mélanges. L'argile est de préférence une bentonite ou une hectorite.

Les argile organophiles sont des argiles modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Elementis, Tixogel VP par la société United catalyst, Claytone 34, Claytone 40, Claytone XL par la société Southern Clay; les stéaralkonium bentonites telles que celles vendues sous les dénominations Bentone 27V par la société Elementis, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées hydrophiles peuvent être obtenues par hydrolyse à haute température d'un composé volatil du silicium dans une flamme oxhydrique, produisant une silice finement divisée. Les silices hydrophiles présentent un nombre important de groupements silanol à leurs surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Les silices pyrogénées hydrophobes peuvent être obtenues par modification de la surface de la silice par une réaction chimique générant une diminution du nombre de groupes silanols, ces groupes pouvant être notamment substitués par des groupements hydrophobes.

Les groupements hydrophobes peuvent être :

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.

- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

Les organopolysiloxanes élastomériques sont en général partiellement ou totalement réticulés et éventuellement de structure tridimensionnelle.

Les organopolysiloxanes élastomères associés à une phase grasse se présentent généralement sous forme de gel constitué d'un organopolysiloxane élastomère associé à une phase grasse, inclus dans au moins une huile hydrocarbonée et/ou une huile siliconée. Ils peuvent être choisis notamment parmi les polymères réticulés décrits dans la demande EP-A-0295886.

Selon cette demande, les organopolysiloxanes élastomères sont obtenus par réaction d'addition et de réticulation d'au moins :

(a) un organopolysiloxane ayant au moins deux groupes alcényle inférieurs par molécule ;
(b) un organopolysiloxane ayant au moins deux atomes d'hydrogène liés à un atome de silicium par molécule ; et
(c) et un catalyseur du type platine.

Les organopolysiloxanes élastomériques associés à une phase grasse peuvent être choisis aussi parmi ceux décrits dans le brevet US-A-5266321, notamment parmi les polyorganopolysiloxanes comprenant des motifs $R_2SiO$ et $RSiO_{1,5}$ et éventuellement des motifs $R_3SiO_{0,5}$ et/ou $SiO_2$ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs $R_2SiO$ sur les motifs $RSiO_{1,5}$ varie de 1/1 à 30/1 ;

Selon un mode de réalisation préféré, l'agent épaississant thixotrope, est choisi parmi les argiles modifiées organophiles telles que l'hectorite modifiée par le stéarate de benzyl di methyl ammonium .

Agent épaississant additionnel

La composition selon l'invention peut comprendre, en outre, un agent épaississant additionnel différent des agents épaississants thixotropes décrits précédemment.

Cet agent épaississant additionnel n'est pas apte à lui seul à rendre à la composition le caractère thixotrope (épaississant non thixotrope) ; il permet notamment d'ajuster la viscosité de la composition pour obtenir un écoulement homogène.

L'agent épaississant additionnel peut être choisi, selon le milieu cosmétiquement acceptable de la composition, parmi :

- les agents épaississants hydrophiles tes que la gomme de guar, la gomme de guar quaternisée, les gommes de guar non-ioniques, les gommes de xanthane, de caroube, de scléroglucane, de gellane, de rhamsan, de karaya, les alginates, la maltodextrine, l'amidon et ses dérivés, l'acide hyaluronique et ses sels,

- les polymères poly(métha)crylates de glycéryle
- la polyvinylpyrrolidone,
- l'alcool polyvinylique,
- les polymères et les copolymères réticulés d'acrylamide,
- les polymères associatifs et notamment les polyuréthanes associatifs,

- les agents épaississants organophiles comme :

  - les gommes de guar alkylées (avec groupe alkyle en $C_1$-$C_6$), telles que celles décrites dans EP-A-708114 ;
  - les polymères gélifiant d'huile comme les polymères tribloc ou en étoile résultant de la polymérisation ou copolymérisation d'au moins monomère à groupe éthylénique, comme les polymères vendus sous la dénomination Kraton ;
  - les résines de polyamides comprenant des groupes alkyles ayant de 12 à 22 atomes de carbone, telles que celles décrites dans US-A-5783657.

- les agent épaississant hydrophobes tels que les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.

L'agent épaississant additionnel peut être présent en une teneur allant de 0,1 % à 20 % en poids, par rapport au poids total de la composition, et de préférence allant de 0,1 % à 10 % en poids.

<u>Milieu solvant organique</u>

La composition selon l'invention peut comprendre un milieu solvant organique comprenant au moins un solvant organique choisi parmi :

- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels l'acétate d'éthyle, l'acétate de butyle, l'acétate de méthyle, l'acétate de propyle, l'acétate d'isopropyle, , l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tel que le carbonate de propylène, le carbonate de diméthyle ,
- les acétals tels que méthylal ;
- et leurs mélanges.

Le milieu solvant organique peut représenter de 30 à 97 % en poids et notamment de 50 à 95 % en poids par rapport au poids total de la composition.

La composition selon l'invention peut comprendre un milieu aqueux.

La teneur en milieu aqueux de la composition peut aller de 5 à 95 % en poids par rapport au poids total de la composition, de préférence de 50 à 70% en poids.

<u>Polymère filmogène</u>

La composition comprend avantageusement au moins un polymère filmogène .

Selon la présente invention, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges.

Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines arylsulfonamide époxy ou encore les résines éthyl tosylamide.

Comme polymère filmogène, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résine toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre. De tels polymères sont décrits par exemple dans les documents EP 1411069 ou WO04/028488.

Le polymère filmogène peut être présent dans la composition selon l'invention en une teneur en matières sèches allant de 0,1 % à 60 % en poids, par rapport au poids total de la composition, de préférence allant de 2 % à 40 % en poids, et mieux de 5 % à 25 % en poids.

Agent auxiliaire de filmification

Pour améliorer les propriétés filmogènes de la composition de vernis à ongles un agent auxiliaire de filmification peut être prévu.

Un tel agent auxiliaire de filmification peut être choisi parmi tous les composés connus de l'homme du métier, comme étant susceptibles de remplir la fonction recherchée, et être notamment choisi parmi les agents plastifiants et les agents de coalescence du ou des polymère(s) filmogène(s).

Ainsi, la composition peut comprendre, en outre, au moins un agent plastifiant et/ou un agent de coalescence. En particulier, on peut citer, seuls ou en mélange, les plastifiants et agents de coalescence usuels, tels que :

- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les esters de glycol ;
- les dérivés de propylène glycol et en particulier le propylène glycol phényléther, le propylène glycol diacétate, le dipropylène glycol éthyléther, le tripropylène glycol méthyléther et le diéthylène glycol méthyléther, le propylène glycol butyléther ;
- des esters d'acides, notamment carboxyliques, tels que des citrates, des phtalates, des adipates, des carbonates, de tartrates, des phosphates, des sébaçates ;
- des dérivés oxyéthylénés, tels que les huiles oxyéthylénées, notamment les huiles végétales, telles que l'huile de ricin ; et
- leurs mélanges.

Le type et la quantité d'agent plastifiant et/ou de coalescence peuvent être choisis par l'homme du métier sur la base de ses connaissances générales.

Par exemple, la teneur en agent plastifiant et/ou de coalescence peut aller de 0,01 % à 20% et en particulier de 0,5 % à 10 % en poids par rapport au poids total de la composition.

Agent d'étalement

Selon un mode de réalisation, la composition selon l'invention comprend un agent d'étalement choisi destiné à favoriser l'application de la composition sur les ongles. Il peut être choisi parmi les huiles de silicones linéaires ou cycliques, notamment celles ayant une viscosité $\leq 6$ centistokes ($6.10^{-6}$ m$^2$/s), et ayant notamment de 3 à 6 atomes de silicium, ces silicones comportant éventuellement un ou plusieurs groupes alkyles ou alkoxy ayant de 1 ou 2 atomes de carbone. Comme huile de silicone utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'agent d'étalement peut représenter de 0,1 à 15% en poids par rapport au poids total de la composition, de préférence de 0,5 à 10% en poids et mieux de 1 à 5% en poids.

Matière colorante

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 60 % en poids, par rapport au poids de la composition, de préférence de 1 % à 40 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Les colorants hydrosolubles sont par exemple le jus de betterave, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyllysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Autres Additifs

La composition peut comprendre, en outre, d'autres ingrédients utilisés couramment dans les compositions cosmétiques. De tels ingrédients peuvent être choisis parmi les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

**Procédé**

Selon un autre aspect, l'invention a pour objet un procédé cosmétique de maquillage des ongles consistant à abaisser la viscosité d'une composition de vernis à ongles présentant une viscosité à 25°C d'au moins 0,6 Pa.s à l'aide d'une action non chimique, simultanément ou préalablement à l'application sur les ongles de ladite composition

Selon un mode de réalisation, le procédé consiste à appliquer à la composition thixotrope sous forme de gel conditionnée dans un récipient, une action non chimique, en particulier mécanique, de manière à fluidifier et réduire la viscosité de ladite composition et permettre son application sur les ongles. Lorsque l'application l'action cesse, la composition dans son conditionnement, après un temps de repos, recouvre sa texture initiale de gel.

Selon un autre mode de réalisation, le procédé consiste à prélever un échantillon de la composition thixotrope dans son conditionnement, puis à appliquer audit échantillon l'action non chimique de manière à fluidifier ladite composition simultanément à son application sur les ongles.

L'action non chimique peut être choisie parmi les actions thermiques tels que par exemple une source de chaleur, les actions mécaniques tel qu'un objet via lequel on applique une contrainte mécanique ou cisaillement à la composition, et leurs associations.

En particulier, cet objet peut être un applicateur se présentant sous forme d'un pinceau, d'une spatule ou d'un embout. De préférence, l'action non chimique est une action mécanique.

La composition de vernis à ongle de l'invention peut être conditionnée dans un récipient délimitant au moins un compartiment, ledit récipient étant fermé par un élément de fermeture.

Le récipient peut être sous toute forme adéquate et peut être, au moins pour partie, en un matériau tel que le verre. Toutefois, des matériaux autres que le verre peuvent être utilisés comme les matériaux thermoplastiques tels que le PP ou le PE ou comme un métal.

L'élément de fermeture peut être couplé au compartiment par vissage en position fermée du récipient. Alternativement, le couplage entre l'élément de fermeture et le récipient peut se faire autrement que par vissage, notamment par encliquetage.

Le récipient peut être associé à un applicateur pouvant être sous forme d'un pinceau constitué d'au moins une touffe de poils. Alternativement, l'applicateur se présente sous forme autre qu'un pinceau, par exemple sous forme d'une spatule ou d'un embout en mousse.

Les exemples qui suivent illustrent de manière non limitative l'invention. Sauf indication contraire, les quantités indiquées sont des pourcentages massiques.

L'invention est illustrée plus en détail dans les exemples suivants. Sauf indication contraire, les quantités sont données en pourcentage en poids par rapport au poids total de la composition.

## Exemples 1 : Vernis à ongles coloré

On prépare un vernis à ongles ayant la composition suivante (% en poids) :

| | Exemple 1 |
|---|---|
| Nitrocellulose à 30 d'alcool isopropylique (viscosité: E22 - 1/2 S) | 5,55 |
| Nitrocellulose à 30 % d'alcool isopropylique (IDYL E27 de Bergerac) | 12,12 |
| Nitrocellulose à 30 % d'alcool isopropylique (Azur E80 de Bergerac) | 0,08 |
| Résine alkyde glycérophtalique ésterifiée par acides gras ramifiés dans l'acétate d'éthyle à 70% (Beckosol ODE 230 70E de Dainippon Ink and Chemicals) | 15,50 |
| Alcool isopropylique | 1,14 |
| Cyclopentadiméthylsiloxane (DC245 fluid de Dow Corning) | 2 |
| Polydiméthylsiloxane 5 cSt (DC200 fluid de Dow Corning) | 0,44 |
| Hectorite modifiée stéaryl benzyl diméthyl ammonium (BENTONE 27 V d'Elementis) | 2,56 |
| Silice pyrogénée hydrophile (Aérosil 200 de Degussa) | 0,46 |
| Laque Red 7 | 0,02 |
| Mica oxyde de titane (Timiron Super Silk MP1005 de Merck) | 0,55 |
| Mica oxyde de titane (Flamenco Red 420 C de Engelhard) | 0,2 |
| Oxychlorure de bismuth | 0,78 |
| Acétate d'éthyle | 19,27 |
| Acétyl citrate de tributyle | 7,54 |
| Acétate de butyle | Qsp 100 |
| Acide citrique, 1 H2O | 0,1 |

La composition présente une viscosité à 25°C de 0,820 Pa.s et est conditionnée dans un pot.

On applique à la composition une agitation mécanique au moyen d'un applicateur, la composition se fluidifie puis on applique au moyen de l'applicateur la composition fluidifiée sur les ongles. On obtient un film brillant, bien couvrant sur les ongles.

Au bout de quelques secondes, le vernis à ongles retrouve sa texture initiale (viscosité proche de la viscosité initiale).

## Exemples 2 : Vernis à ongles non coloré

| | Exemple 2 |
|---|---|
| Nitrocellulose à 30 d'alcool isopropylique (viscosité: E22 - 1/2 S) | 5,2 |
| Nitrocellulose à 30 % d'alcool isopropylique (IDYL E27 de Bergerac) | 13,70 |
| Résine alkyde glycérophtalique éesterifiée par acides gras ramifiés dans l'acétate d'éthyle à 70% (Beckosol ODE 230 70E de Dainippon Ink and Chemicals). | 16,19 |
| Alcool isopropylique | 0,99 |
| Polydiméthylsiloxane 5 cSt (DC200 fluid de Dow Corning) | 0,5 |
| Hectorite modifiée stéaryl benzyl diméthyl ammonium (BENTONE 27 V d'Elementis) | 2,8 |
| Silice pyrogénée hydrophile (Aérosil 200 de Degussa) | 0,526 |
| Acétate d'éthyle | 19,94 |
| Acétyl citrate de tributyle | 7,96 |
| Acétate de butyle | Qsp 100 |
| Acide citrique, 1 H2O | 0,1 |

## Exemples 3 : Vernis à ongles

### a) Synthèse d'un polycondensat pentaérythrityl benzoate/isophtalate/isostéarate

Dans un réacteur équipé d'une agitation mécanique, d'une arrivée d'argon et d'un système de distillation, on charge 227,5 g d'acide benzoïque, 72,8 g d'acide isostéarique et 118,3 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 18 heures. On refroidit à une température comprise entre 100 et 130°C puis on introduit 91 g d'acide isophtalique et on chauffe à nouveau progressivement jusqu'à 220°C pendant environ 11 heures.

On obtient ainsi 430 g de polycondensat pentaérythrityl benzoate/isophta-late/isostéarate sous la forme d'une huile épaisse qui se solidifie à température ambiante.

Le polycondensat présente les caractéristiques suivantes :

- Indice d'acide = 12,7
- Indice d'hydroxyle = 49
- $\eta_{110°C}$ = 25,4 Poises (soit 2540 mPa.s)
- rapport entre le nombre de mole d'acide monocarboxylique aromatique et le nombre de mole d'acide monocarboxylique non aromatique : 7,28.

On prélève 420g de polycondensat obtenu ci-dessus, on le chauffe à 100-120°C et on coule lentement 180g d'acétate de butyle sous agitation puis on clarifie par filtration à chaud sur fritté n°2.

On obtient après refroidissement à température ambiante 600 g de solution de polycondensat à 70% dans l'acétate de butyle, se présentant sous la forme d'un liquide visqueux jaune pâle et possédant une viscosité à 25°C d'environ 800 centipoises (mPa.s)

b) on prépare le vernis à ongles suivant :

| | |
|---|---|
| Nitrocellulose à 30 d'alcool isopropylique (viscosité: E22 - 1/2 S) | 4,84 |
| Nitrocellulose à 30 % d'alcool isopropylique (IDYL E27 de Bergerac) | 12 |
| Nitrocellulose à 30 % d'alcool isopropylique (Azur E80 de Bergerac) | 0,08 |

(suite)

| | |
|---|---|
| Résine alkyde glycérophtalique ésterifiée par acides gras ramifiés dans l'acétate d'éthyle à 70% (Beckosol ODE 230 70E de Dainippon Ink and Chemicals) | 2,45 |
| Solution à 70% en matières sèches du polymère synthétisé au a) dans l'acétate de butyle | 11,49 |
| Alcool isopropylique | 1,4 |
| Cyclopentadiméthylsiloxane (DC245 fluid de Dow Corning) | 2 |
| Polydiméthylsiloxane 5 cSt (DC200 fluid de Dow Corning) | 0,44 |
| Hectorite modifiée stéaryl benzyl diméthyl ammonium (BENTONE 27 V d'Elementis) | 3,19 |
| Silice pyrogénée hydrophile (Aérosil 200 de Degussa) | 0,37 |
| Laque Red 7 | 0,02 |
| Mica oxyde de titane (Timiron Super Silk MP1005 de Merck) | 0,55 |
| Mica oxyde de titane (Flamenco Red 420 C de Engelhard) | 0,2 |
| Oxychlorure de bismuth | 0,78 |
| Acétate d'éthyle | 15 |
| Acétyl citrate de tributyle | 7,37 |
| Acétate de butyle | Qsp 100 |
| Acide citrique, 1 H2O | 0,13 |

La composition présente une viscosité à 25°C, mesurée au Rhéomat 180, de 0,820 Pa.s

On applique à la composition une agitation mécanique au moyen d'un applicateur, la composition se fluidifie puis on applique au moyen de l'applicateur la composition fluidifiée sur les ongles. On obtient un film brillant, bien couvrant sur les ongles.

Au bout de quelques secondes, le vernis à ongles retrouve sa texture initiale (viscosité proche de la viscosité initiale).

On évalue le comportement thixotrope de la composition via les mesures de viscosité de la composition selon le protocole décrit plus haut.

La composition présente une viscosité, telle que mesurée à l'étape e), de 45 Pa.s.

Elle présente un comportement thixotrope caractérisé par la différence (viscosité mesurée au cours de l'étape c) à une vitesse de cisaillement de 1 $s^{-1}$ - viscosité mesurée au cours de l'étape e) à une vitesse de cisaillement de 1 $s^{-1}$) de l'ordre de 100 Pa.s.

Cette composition présente un module de rigidité plateau Gp compris entre 1000 et 3000 Pa, de l'ordre de 2000 Pa, une élasticité $\delta_p$ allant de 20°, et un seuil d'écoulement $\tau_c$ de 100 Pa.

## Revendications

1.  Composition de vernis à ongles comprenant un milieu cosmétiquement acceptable et au moins un agent épaississant thixotrope, ladite composition présentant une viscosité à 25°C d'au moins 0,6 Pa.s.

2.  Composition selon la revendication 1, **caractérisée en ce qu'**elle présente une viscosité allant de 0,6 à 20 Pa.s, de préférence de 0,7 à 15 Pa.s et, mieux de 0,75 à 10 Pa.s.

3.  Composition de vernis à ongles comprenant un milieu cosmétiquement acceptable et au moins un agent épaississant thixotrope, ladite composition présentant un caractère thixotrope tel que la différence de viscosité, mesurée selon le protocole indiqué dans la description, à une vitesse de cisaillement de 1 $s^{-1}$ : (viscosité mesurée au cours de l'étape c) - viscosité mesurée au cours de l'étape e)) est supérieure ou égale à 1 Pa.s.

4.  Composition selon la revendication 3, **caractérisée en ce que** la différence de viscosité (viscosité mesuré au cours de l'étape c) - viscosité mesurée au cours de l'étape e)) est supérieure ou égale à 10 Pa.s, mieux d'au moins 20 Pa.s, encore mieux d'au moins 30 Pa.s, préférentiellement d'au moins 40 Pa.s..

5.  Composition selon la revendication 3 ou 4, **caractérisée en ce que** la différence de viscosité (viscosité mesurée au cours de l'étape c) - viscosité mesurée au cours de l'étape e)) va de 1 à 1000 Pa.s, mieux de 20 à 500 Pa.s, en particulier de 40 à 200 Pa.s.

6.  Composition de vernis à ongles comprenant un milieu cosmétiquement acceptable et au moins un agent épaississant thixotrope, ladite composition présentant un module de rigidité plateau Gp supérieur à 100 Pa.

7. Composition selon la revendication 6, **caractérisée en ce que** le module de rigidité plateau Gp va de 100 à $2.10^6$ Pa.s, de préférence de $5.10^2$ à $10^4$ Pa.s, mieux de 800 à 4000 Pa.s, et en particulier de 1000 à 3000 Pa.s.

8. Composition selon l'une des revendications précédentes, **caractérisée en ce que** l'agent épaississant thixotrope est choisi parmi les argiles hydrophiles ou organophiles, les silices pyrogénées hydrophiles ou hydrophobes, les organopolysiloxanes élastomériques et leurs mélanges.

9. Composition selon l'une quelconque des revendications précédentes, **caractérisée en ce que** l'agent épaississant thixotrope est choisi parmi argiles modifiées organophiles telles que l'hectorite modifiée par le stéarate de benzyl di methyl ammonium.

10. Composition selon l'une quelconque des revendications précédentes, **caractérisée en ce que** l'agent épaississant thixotrope comprend en outre une silice pyrogénée hydrophobe.

11. Composition selon l'une quelconque des revendications précédentes, **caractérisée en ce que** l'agent épaississant thixotrope est présent en une teneur allant de 1,7 % à 15 % en poids, par rapport au poids total de la composition, de préférence allant de 2 % à 10% en poids, préférentiellement allant de 2 % à 7,5 % en poids.

12. Composition selon l'une quelconque des revendications précédentes, **caractérisée en ce qu'**elle comprend au moins un polymère filmogène.

13. 12 8, **caractérisée en ce que** le polymère filmogène est présent en une teneur allant de 0,1 % à 60 % en poids, par rapport au poids total de la composition, de préférence allant de 2 % à 40 % en poids, et mieux de 5 % à 25 % en poids.

14. Composition selon l'une quelconque des revendications précédentes, **caractérisée en ce qu'**elle comprend au moins solvant organique.

15. Composition selon la revendication précédente, **caractérisée en ce que** le solvant organique représente de 30 à 97% en poids et notamment de 50 à 95% en poids par rapport au poids total de la composition.

16. Composition selon l'une quelconque des revendications précédentes, **caractérisée en ce qu'**elle comprend au moins un agent d'étalement choisi parmi les huiles de silicones linéaires ou cycliques, notamment ayant une viscosité $\leq 6$ centistokes ($6.10^{-6}$ m$^2$/s).

17. Composition selon la revendication 16, **caractérisée en ce que** l'agent d'étalement représente de 0,1 à 15% en poids par rapport au poids total de la composition, de préférence de 0,5 à 10% en poids et mieux de 1 à 5% en poids

18. Composition selon l'une quelconque des revendications précédentes, **caractérisée en ce qu'**elle comprend au moins matière colorante choisie parmi les colorants hydrosolubles, les pigments, les nacres, les paillettes et leurs mélanges.

19. Composition selon la revendication précédente, **caractérisée en ce que** les matières colorantes sont présentes en une teneur allant de 0,01 % à 60 % en poids, par rapport au poids de la composition, de préférence de 1 % à 40 % en poids.

20. Utilisation d'une composition selon l'une des revendications 1 à 19, pour obtenir un film de vernis déposé sur les ongles, présentant de bonnes propriétés de brillance et/ou une bonne couvrance.

21. Utilisation d'une composition selon l'une des revendications 1 à 19, pour obtenir un film de vernis déposé sur les ongles qui sèche rapidement.

22. Procédé cosmétique de maquillage des ongles consistant à abaisser la viscosité d'une composition de vernis à ongles présentant une viscosité à 25°C d'au moins 0,6 Pa.s à l'aide d'une action non chimique, simultanément ou préalablement à l'application sur les ongles de ladite composition.

**23.** Procédé selon la revendication 22, **caractérisé en ce que** ladite action non chimique est choisie parmi les actions thermiques, mécaniques et leurs associations.

**24.** Procédé selon la revendication 22 ou 23, **caractérisé en ce que** l'action non chimique est une action mécanique.

**25.** Procédé selon l'une des revendications 24, **caractérisé en ce que** l'action mécanique est appliquée via un applicateur.

**26.** Procédé selon l'unes des revendications 22 à 25, **caractérisé en ce que** le vernis présente une viscosité allant de 0,6 à 20 Pa.s, de préférence de 0,7 à 15 Pa.s et, mieux de 0,75 à 10 Pa.s.

**27.** Procédé selon l'une des revendications 22 à 26, **caractérisé en ce que** la composition de vernis comprend au moins un agent épaississant thixotrope.

**28.** Procédé selon l'une des revendications 22 à 27, **caractérisé en ce** l'agent épaississant thixotrope est choisi parmi les argiles hydrophiles ou organophiles, les silices pyrogénées hydrophiles ou hydrophobes, les organopolysiloxanes élastomériques et leurs mélanges.

**29.** Procédé selon l'une des revendications 22 à 28, **caractérisé en ce** l'agent épaississant est choisi parmi les argiles modifiées organophiles telles que l'hectorite modifiée par le stéarate de benzyl di methyl ammonium.

**30.** Procédé selon l'une quelconque des revendications 22 à 29, **caractérisé en ce que** l'agent épaississant thixotrope comprend en outre une silice pyrogénée hydrophobe.

**31.** Procédé selon l'une des revendications 22 à 30, **caractérisé en ce** l'agent épaississant thixotrope est présent en une teneur allant de 1,7 % à 15 % en poids, par rapport au poids total de la composition, de préférence allant de 2 % à 10% en poids, préférentiellement allant de 2 % à 7,5 % en poids.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0295886 A
- US 5266321 A
- EP 708114 A
- US 5783657 A
- EP 898958 A
- EP 1411069 A
- WO 04028488 A

**Littérature non-brevet citée dans la description**

- **P. COUSSOT ; J.L. GROSSIORD.** Comprendre la rhéologie - De la circulation du sang à la prise du béton. *EDP Science,* 2002, 16, 17
- **G. COUARRAZE ; J.L. GROSSIORD.** Initiation à la rhéologie. 1991